(19) Europäisches Patentamt
European Patent Office
Office européen des brevets

(11) Numéro de publication : **0 399 913 B1**

(12) # FASCICULE DE BREVET EUROPEEN

(45) Date de publication du fascicule du brevet : **22.09.93 Bulletin 93/38**

(21) Numéro de dépôt : **90401390.1**

(22) Date de dépôt : **23.05.90**

(51) Int. Cl.5 : **C07C 47/02,** C07C 31/02, C07C 27/20, C07C 29/16, C07C 45/50, B01J 12/00, H05H 1/24

(54) **Procédé de synthèse de produits "oxo" par voie plasma et installation comprenant un réacteur à plasma utile dans le procédé.**

(30) Priorité : **24.05.89 FR 8906799**

(43) Date de publication de la demande : **28.11.90 Bulletin 90/48**

(45) Mention de la délivrance du brevet : **22.09.93 Bulletin 93/38**

(84) Etats contractants désignés : **AT BE CH DE DK ES GB GR IT LI LU NL SE**

(56) Documents cités :
**EP-A- 0 292 391**
**FR-A- 2 593 493**
**FR-A- 2 622 893**

(73) Titulaire : **ELECTRICITE DE FRANCE Service National**
**2, rue Louis Murat**
**F-75008 Paris (FR)**

(72) Inventeur : **Doubla, Avaly**
**36, Avenue de la Division Leclerc**
**F-94230 Cachan (FR)**
Inventeur : **Amouroux, Jacques Eugène**
**13b, rue de Villezien**
**F-91440 Bures/Yvette (FR)**
Inventeur : **Brisset, Jean-Louis**
**4, rue des Ecoles**
**F-75005 Paris (FR)**

(74) Mandataire : **Martin, Jean-Jacques et al**
**Cabinet REGIMBEAU 26, Avenue Kléber**
**F-75116 Paris (FR)**

EP 0 399 913 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un procédé de synthèse de produits hydrocarbonés du type "oxo", c'est-à-dire présentant une substitution avec un groupe carbonyle, ou des produits dérivés ayant comme substitution des groupes hydroxyles résultant de la réduction des groupes carbonyles. Cette synthèse de produits "oxo" est obtenue en faisant réagir un substrat hydrocarboné insaturé avec un mélange de monoxyde de carbone et d'hydrogène. En particulier, la présente invention concerne la synthèse de produits "oxo" ou leurs dérivés obtenus en faisant réagir un alcène avec un mélange de monoxyde de carbone et d'hydrogène. La présente invention concerne également des installations comprenant un réacteur à plasma utiles dans le procédé. La réaction d'un substrat hydrocarboné insaturé avec un mélange de monoxyde de carbone et d'hydrogène conduit à la synthèse d'un aldéhyde par fixation d'un groupe

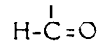

sur la double liaison de l'alcène, mais on obtient également des alcools car cette fonction aldéhyde est réduite en fonction alcool

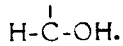

On obtient également des produits dits d'aldocondensation résultant de la condensation de deux aldéhydes pour donner un aldol ou hydroxyaldéhyde, ou un aldéhyde insaturé résultant de la déshydratation dudit aldole. La présence d'eau résultant de cette déshydratation permet également que le mélange de produits obtenus dans la réaction contienne des acides carboxyliques ou des esters. Dans la présente demande, on entend donc par produits dérivés des produits "oxo", des alcools d'une part, et d'autre part les produits dérivant de l'aldocondensation, à savoir notamment des aldols, des aldéhydes insaturés, des acides carboxyliques et des esters.

Pour préparer ces produits, plusieurs techniques sont connues, chacune utilisant des variantes plus ou moins spécifiques (température, pression, catalyseur, etc). Ces produits sont en général obtenus dans des conditions assez sévères, notamment une température élevées de 100 à 200°C, des pressions élevées 150 à 200 atm et enfin la présence de catalyseurs spécifiques et actifs tels que Cu, Ni, Co, Rh, Ru, etc. En outre, la cinétique des réactions est en général assez lente et la durée de traitement de plusieurs heures.

Le but de la présente invention est de fournir un procédé de synthèse de produits "oxo" ou produits dérivés qui se déroule dans des conditions plus douces et donc plus économiques, notamment en utilisant des matières premières moins onéreuses, avec un rendement et une vitesse de réaction améliorés.

Conformément à la présente invention, ce but est atteint en ayant recours à une réaction en phase plasma.

Plus précisément, la présente invention a pour objet un procédé de synthèse de produits "oxo" ou leurs dérivés consistant en des composés hydrocarbonés présentant des groupes carbonyles et/ou hydroxyles, et notamment des groupes carboxylés, caractérisé en ce qu'on fait réagir un substrat hydrocarboné présentant une insaturation carbonée tel qu'un alcène avec des réactifs consistant en un mélange d'hydrogène et de monoxyde de carbone, la réaction ayant lieu suite à la mise en contact du substrat avec les espèces activées neutres du mélange de réactifs activé à l'état de plasma.

Les espèces réactives de $H_2$ et CO sont excitées électroniquement par le plasma. En particulier un carbène(:CO) qui, compte tenu de ses propriétés acido-basiques, conduit à sa fixation sur les sites insaturés du substrat hydrocarboné, notamment un alcène, ou sur ceux d'un produit de craquage dudit substrat. Le carbène(:CO) compte tenu de ses propriétés radicalaires peut en effet être responsable du craquage du substrat hydrocarbure.

On obtient ainsi dans des conditions très douces la synthèse de produits "oxo", à savoir des aldéhydes résultant de la fixation du groupe

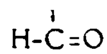

sur l'insaturation carbonée C=C du substrat insaturé tel que la double liaison de l'alcène mais également les produits dérivés de ces produits "oxo", à savoir des alcools résultant de la réduction de la fonction aldéhyde, mais aussi des produits dits d'aldocondensation ou dérivés de ces derniers comme cité précédemment, à savoir des aldols, des aldéhydes insaturés, des acides carboxyliques, des esters, etc.

Le substrat et les réactifs sont introduits dans un réacteur à plasma à la pression atmosphérique à une température moyenne voisine de la température ambiante et sans catalyseur, les espèces réactives étant excitées uniquement par le plasma. Le principe de la méthode est en fait une réaction en catalyse homogène. La vitesse de réaction, à savoir la fixation de CO sur le substrat hydrocarboné tel que l'alcène, est très rapide

(par exemple 4 à 5 min de traitement).

Les avantages du procédé selon la présente invention sont schématisés dans le tableau ci-après.

| Voie classique | Voie plasma |
|---|---|
| Températures élevées<br>100 à 200°C | Température ambiante<br>25 à 30°C |
| Pressions élevées<br>150 à 200 atm | Pression atmosphérique<br>1 atm |
| Catalyseurs spécifiques et actifs<br>(Co, Rh, Ru, Ni, Cu, etc...) | Sans catalyseurs<br>activation due au plasma |
| Cinétique lente<br>Durée de traitement :<br>quelques heures | Cinétique rapide<br>Durée de traitement :<br>5 à 10 min |

On obtient un mélange de différents composés qui peuvent être séparés par toute méthode connue de l'homme de l'art notamment par distillation.

En particulier, selon la présente invention, on peut faire réagir un alcène pour obtenir un mélange comportant l'aldéhyde et l'alcool résultant de la fixation respectivement d'un groupe

$$H-\overset{|}{C}=O$$

ou de son groupe obtenu par réduction

$$H-\overset{|}{C}-OH$$

sur la double liaison de l'alcène, ou pour obtenir l'un seulement de ces deux composants aldéhydes ou alcools, en le séparant du mélange par exemple par distillation.

Comme on l'a vu précédemment, les produits majoritaires obtenus sont des aldéhydes et des alcools, toutefois en faisant varier les différents paramètres de fonctionnement du réacteur, on peut obtenir des taux de formation de ces produits variables. En particulier, si le rapport des débits volumiques $CO/H_2$ est supérieur à 1, on obtiendra de façon optimale les aldéhydes et alcools, de même, en revanche si le rapport des débits volumiques $CO/H_2$ est voisin de 1/2, il y aura production préférentielle des produits dits d'aldocondensation.

Avantageusement, selon l'invention, l'activation du mélange hydrogène-monoxyde de carbone se fait dans un réacteur à plasma, le substrat étant mis en contact avec le mélange activé en dehors de la zone d'activation du plasma. En effet, si le substrat se trouve introduit dans la zone d'activation et donc est lui-même activé, la constante diélectrique du milieu s'en trouve affectée et devient telle que l'établissement d'un champ électrique suffisant pour l'excitation électronique des réactifs et donc pour que la synthèse se produise, nécessite d'avoir recours à un générateur haute tension délivrant une tension beaucoup trop élevée.

Un réacteur à plasma du type décharge couronne pointe-plan, en raison de sa facilité de mise en oeuvre

d'adaption au problème spécifique de la réaction de synthèse oxo en cause, constitue un réacteur de choix dans ce procédé selon l'invention.

Une caractéristique avantageuse de la présente invention consiste alors en ce que l'électrode pointe du réacteur à plasma de type à décharge couronne est disposée dans le réacteur parallèlement à l'électrode plane, et le substrat est introduit dans le réacteur dans l'axe de la pointe en dessous de la zone d'activation située entre la pointe (portée à la haute tension) et le plan (relié à la masse). La configuration ainsi adoptée est une configuration dite parallèle qui présente l'avantage de séparer la réactivité chimique des espèces chargées de celle due aux espèces neutres activées par la décharge qui sont responsables de la synthèse de produits "oxo" tels que les carbène(:CO). Ceci permet également d'obtenir un champ électrique aussi important que possible sans transiter au régime d'arc.

On obtient de meilleurs rendements lorsque l'électrode pointe est chargée positivement et l'électrode plane reliée à la masse.

Selon une autre caractéristique, le monoxyde de carbone est introduit par une électrode creuse constituée par la pointe, l'hydrogène étant introduit par la périphérie du réacteur. Dans ces conditions d'introduction, tout le monoxyde de carbone est activé. Si on l'introduisait par la périphérie du réacteur comme l'hydrogène, seul celui-ci serait activé en quantité suffisante.

La présente invention a également pour objet une installation utile dans le procédé selon l'invention comprenant un réacteur à plasma du type à décharge couronne pointe-plan, caractérisé en ce qu'il comporte une ou plusieurs électrodes pointes creuses par lesquelles le réacteur est alimenté en gaz de monoxyde de carbone, l'électrode pointe étant disposée parallèlement à l'électrode plane, et le substrat hydrocarboné liquide étant placé sous la pointe dans son axe à une distance pointe substrat inférieure à la distance pointe-plan.

Pour travailler à débit important et concevoir une installation industrielle de grand tonnage, une variante du procédé consiste à avoir recours à un réacteur à plasma thermique, notamment du type à torche plasma produite par haute fréquence, le mélange $CO+H_2$ activé à l'état de plasma étant ensuite à une trempe par un lit de particules fluidisées, par un courant gazeux d'hydrogène à une température comprise entre 20 et 150°C, avant d'être mis une fois refroidi en contact avec le substrat, lequel peut se trouver en phase liquide ou en face vapeur.

Le procédé consiste alors à mélanger un plasma thermique $CO+H_2$ dans un lit de particules solides fluidisé par un courant gazeux à une température inférieure à celle du plasma et à faire réagir le mélange activé refroidi ainsi produit avec le substrat hydrocarboné pour former les produits "oxo".

Avantageusement, on fait circuler en aval du lit fluidisé le courant gazeux à travers un réacteur tubulaire dans lequel le plasma réagit avec le substrat circulant à contre courant.

Le lit fluidisé est du type jaillissant.

L'installation pour la mise en oeuvre de ce procédé comprend donc un dispositif à lit fluidisé comportant une enceinte comprenant des moyens d'injection du gaz de fluidisation au niveau de son fond, des moyens de sortie de ce dernier et contenant une masse de particules solides destinées à former un lit fluidisé, et une torche à plasma adaptée pour injecter le plasma à l'intérieur de l'enceinte dans le lit de particules fluidisé. Il est prévu un réacteur tubulaire relié à la sortie de ladite enceinte.

Avantageusement, la torche à plasma est raccordée au niveau d'une paroi latérale de l'enceinte de façon à ce que le plasma soit injecté latéralement dans le lit fluidisé.

Le réacteur tubulaire est avantageusement du type colonne à garnissage dans lequel la réaction se produit, le substrat circulant à contre courant du flux de mélange de $CO+H_2$ activé.

D'autres caractéristiques et avantages de la présente invention apparaîtront à la lumière de la description qui va suivre faite en référence à des dessins sur lesquelles :

<u>La figure 1</u> représente le schéma d'installation d'un réacteur à plasma du type à décharge couronne pointe-plan utile dans le procédé selon l'invention.

<u>Les figures 2 à 5</u> représentent des spectres infrarouges.

<u>La figure 2</u> représente la variation de la transmission en fonction du nombre d'onde de l'hexène-1 traité par le procédé selon l'invention pour deux distance différentes ; a) témoin, b) d=10 mm ; c) $d_2$=17 mm (distance interélectrode).

Les conditions de travail sont :

I = 50 $\mu$A

t = 8,39 minutes

$CO/H_2$ = 1/2.

<u>La figure 3</u> représente la variation de la transmission en fonction du nombre d'onde de l'hexène-1 (témoin) et du produit obtenu après traitement.

Les conditions de travail sont :

$CO/H_2$ = 1/2

I = 50 µA
t = 8,00 minutes
V = 18,5 kV
$d_2$ = 13 mm ; $d_1$ = 7 mm

La figure 4 montre la variation de l'intensité d'absorption de la bande -OH de l'hexène-1 (témoin) et du produit obtenu après traitement.
Les conditions de travail sont :
t = 8,39 minutes
I = 50 µA
V = 16 kV
$d_2$ = 17 mm ; $d_1$ = 8 mm
$CO/H_2$ = 1/2.

La figure 5 représente l'influence de l'intensité du courant sur la nature des produits formés (composés carbonylés et hydroxylés).
Les conditions de travail sont :
t = 10 minutes
V = 18,5 kV
$d_2$ = 10 mm ; $d_1$ = 8 mm
$CO/H_2$ = 1/2
I variable
a) 20 µA ; b) 50 µA ; c) 80 µA ; d) 100 µA.

La figure 6 représente sur l'exemple de l'hexène 1 l'influence de l'intensité du courant sur la nature et le pourcentage des produits principaux obtenus par CPG.
Les conditions de travail sont :
$d_2$ = 13 mm ; $d_1$ = 7 mm
durée t = 8 minutes
$CO/H_2$ = 1 (rapport volumique)

La figure 7 représente sur l'exemple de l'hexène 1 l'influence du rapport $CO/H_2$ sur la nature et le pourcentage des produits principaux obtenus par CPG.
Les conditions de travail sont :
I = 50 µA
V = 18,5 kV
durée t = 8,39 minutes
$d_2$ = 15 mm ; $d_1$ = 8 mm.

La figure 8 représente la variation des taux de formation des produits "oxo" en fonction du rapport $CO/H_2$ (décharge en pointe négative).
Les conditions de travail sont :
I = 50µA.

La figure 9 représente la variation des pourcentage d'hexène-1 non dégradé en fonction du débit de CO, $H_2$ ou CO + $H_2$ (dans le rapport 1/1).
Les conditions de travail sont :
I = 50 µA
t = 10 minutes (décharge en pointe positive).

La figure 10 représente des spectres infrarouges montrant l'influence du rapport $CO/H_2$ sur la nature des produits formés (fonctions carbonylées) ; a) témoin ; b) $CO/H_2$ = 1/2 ; c) $CO/H_2$ = 1.

La figure 11 représente l'influence du rapport $CO/H_2$ sur la nature des produits formés (fonctions hydroxylées).
Les conditions de travail pour l'obtention des spectres sont
I = 50 µA
t = 8,39 minutes
V = 17,5 kV
$d_2$ = 15 mm ; $d_1$ = 8 mm.

La figure 12 représente des spectres infrarouges montrant l'influence de la durée de traitement sur la nature des produits formés (aldéhydes, esters et acides carboxyliques).
Les conditions de travail sont
I = 50 µA
V = 21,5 kV
$d_2$ = 15 mm ; $d_1$ = 8 mm

a) t = 12 minutes ; b) t = 8,39 minutes ; c) t = 4 minutes.

La figure 13 représente un schéma de l'installation comportant un dispositif lit fluidisé.

Le choix d'un substrat hydrocarboné insaturé du type alcène, à savoir l'hexène-1 soumis à une décharge éléctrique en l'occurence à une décharge couronne dans un mélange adéquat de CO et de $H_2$ avec des rapports $CO/H_2$ variables, a permis de préparer dans des conditions très douces des produits "oxo" (aldéhyde, alcool, acide carboxylique, ester, etc).

On a montré en effet qu'il est possible de synthétiser des produits "oxo" par des procédés utilisant la voie plasma d'une part, et d'autre part on a pu déterminer les paramètres prépondérants permettant de contrôler cette production, à savoir quels sont les produits majoritaires et comment varient les taux de formation de ces produits en fonction des différents paramètres du fonctionnement du réacteur.

L'expérience a été entreprise dans un réacteur chimique à plasma du type à décharge couronne pointe-plan à la pression atmosphérique. Le schéma de l'installation est représentée à la figure 1. Elle comprend globalement deux parties :

- un générateur haute tension, délivrant dans notre cas particulier une tension maximale continue de 30 kV, et
- le réacteur chimique constitué d'un tube en quartz dans le cas présent de 2,80 cm de diamètre intérieur.

Les débits d'alimentation en CO et $H_2$ sont contrôlés par des débitmètres massiques.

la zone réactionnelle est comprise entre la pointe portée à la haute tension, le plan relié à la masse et les parois du tube en quartz.

Les substrats liquides, à savoir en l'espèce l'hexène-1, sont introduits dans le réacteur par l'intermédiaire d'un porte-échantillon, disposé dans l'axe de la pointe et placé à une distance de la pointe variant dans le cas présent entre 7 et 10 mm. La pointe est disposée parallèlement à l'électrode constituée par le plan qui est porté à la masse.

Plus précisément, les conditions de travail à l'aide d'un appareillage tel que représenté à la figure 1 dans la description qui va suivre ont été les suivantes :

- pointe portée à la haute tension (+/-),
- gaz plasmagènes : CO + $H_2$ dans des proportions variables,
- d1 (distance pointe-substrat) : 7 à 10 mm,
- d2 (distance pointe-plan) : 10 à 25 mm,
- intensité du courant : 20 à 70 $\mu$A,
- tension d'alimentation : 10 à 25 kV,
- substrat utilisé : hexène-1 : $CH_3$-$(CH_2)_3$-CH = $CH_2$,
- techniques d'analyses : IR, CPG et spectroscopie Raman.

1) Principe de la réaction

La synthèse de produits "oxo" en phase plasma peut être considérée comme une réaction procédant en deux étapes :

- une première étape est l'activation des réactifs (CO et $H_2$) entrant dans le réacteur. Cette activation produit des espèces tant radicalaires que moléculaires douées de grandes réactivités chimiques. Elle est importante et croît avec l'intensité du courant injecté dans la décharge et le champ électrique résultant ;
- une deuxième étape consiste en la réaction des espèces activées avec le substrat (hexène-1), selon une réaction acido/basique ou addition nucléophile et/ou électrophile, réaction facilitée par les orbitales frontières vides de la liaison insaturée de l'hexène-1.

Les paramètres de travail qui ont été étudiés sont ceux relatifs au fonctionnement du réacteur, à savoir l'intensité du courant, la durée du traitement du substrat et le débit relatif en $H_2$ et CO (rapport $CO/H_2$ variable).

2) Conditions opératoires

Le réacteur est lavé à l'acétone afin de le débarrasser des dépôts de noir de carbone qui se sont fixés sur les parois du réacteur et sur les électrodes. Il est ensuite purgé à l'argon. L'hexène-1 est introduit par l'intermédiaire du porte-échantillons et des débits de CO et $H_2$ sont contrôlés par des débitmètres massiques. Le CO est introduit par l'électrode creuse constituée par la pointe et l'hydrogène par la périphérie du réacteur. L'expérience est effectuée à la pression atmosphérique. La décharge électrique est amorcée et maintenue à la puissance choisie pendant toute la durée de l'expérience.

L'analyse des produits après réaction est effectuée par spectrométrie IR à transformée de Fourier, afin de déterminer les groupements fonctionnels résultant de la réaction et par chromatographie gazeuse afin de déterminer l'ossature carbonée des produits obtenus. L'établissement d'un parallèle entre les résultats obtenus

par IR et ceux obtenus par CPG, nous permet de suivre l'évolution des différents produits de la décharge et d'interpréter le rôle de certains paramètres.

Dans le but d'apprécier les modifications chimiques apportées par la décharge, l'analyse des produits résultant de la réaction ainsi que celui du témoin a été effectuée par spectrométrie infrarouge. Le produit et le témoin sont enregistrés sur le même spectre, voir par exemple figure 2.

- Le spectre IRTF (IR à transformée de Fourier) de l'hexène-1 témoin présente des bandes d'absorption à 2920-2840 $cm^{-1}$ 1610-1650 $cm^{-1}$ et
138-1410 $cm^{-1}$, qui sont caractéristiques des liaisons C-H, C=C et C-C.

Le spectre de l'hexène-1 traité présente quant à lui des bandes d'absorption supplémentaires très intenses. Ainsi, on observe des bandes à 3404 $cm^{-1}$, 2920-2840 $cm^{-1}$, 1640 $cm^{-1}$ (moins intense), 1683-1720 $cm^{-1}$ qui sont propres aux liaisons O-H ($H_2O$ et/ou alcools), C-H, C=C, C=O et

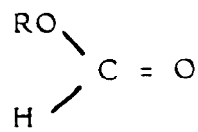

Les liaisons chimiques que l'on pourrait attribuer à ces bandes sont rassemblées dans le tableau III.

| limite d'absorption en ($cm^{-1}$) | liaison | Intensité | Composés ou groupements fonctionnels |
|---|---|---|---|
| 3200-3600 | O-H | forte et large | $H_2O$ ou R-OH primaire ou secondaire |
| 2500-3560 | O-H | forte et large | Acides carboxyliques |
| 3400 | O-H associée | forte et large | Alcools (dimères) ou aldols |
| 3200-3300 | O-H | forte et large | |
| 1683-1720 | C=O | forte | Cétones, acides carboxyliques, esters non saturés |
| 1640 | C=C | forte | C=C conjuguée avec une liaison C-C, ou C=O |
| 1640 | O-H | forte | H-O-H |
| 1280-1480 | C-O | forte | Acides carboxyliques, esters, alcools |

3) <u>Influence de l'intensité du courant sur la nature des produits formés</u>

On a fait varier l'intensité du courant dans une gamme allant de 30 à 100 μA, les autres paramètres étant maintenus constants : durée de la décharge = 8 min, distance (pointe-plan) = 17 mm, distance (pointe-substrat) = 8 mm, débit de CO = 0,10 l/min, débit de $H_2$ = 0,10 l/min.

Le tableau 1 ci-après montre l'évolution des différents produits principaux obtenus en fonction de l'intensité

(analyse effectuée par chromatographie CPG).

Tableau 1

| Conditions % X Produits formés | $I_1$ = 30 µA X % produits | $I_2$ = 50 µA X % produits | $I_3$ = 70 µA X % produits | $I_4$ = 100 µA X % produits |
|---|---|---|---|---|
| Pentène-1 | 1,96 | 1,50 | 1,43 | 1,31 |
| Hexène-1 | 8,91 | 2,16 | 1,20 | 0,85 |
| Heptanal-1 | 15,92 | 18,33 | 14,53 | 11,91 |
| Heptanol-1 | 13,93 | 17,56 | 17,20 | 12,19 |
| Ethyl-laurate | 15,58 | 21,45 | 26,34 | 23,70 |

L'analyse des produits par chromatographie a montré qu'il se forme de l'heptanal-1, de l'heptanol-1 et du laurate d'éthyle comme produits majoritaires. On obtient aussi du pentène-1 comme sous-produit provenant du craquage de l'hexène-1.

L'examen de la figure 6 et du tableau 1 relatifs à l'évolution des produits "oxo" en fonction de l'intensité du courant montre que les taux de formation de l'heptanol-1 et de l'aldéhyde (heptanal-1) augmentent et passent par un maximum puis diminuent progressivement au fur et à mesure que l'intensité du courant augmente. La diminution est beaucoup plus prononcée pour l'aldéhyde que pour l'alcool (heptanol-1).

La courbe relative au produit d'aldocondensation consistant dans le laurate d'éthyle ($C_{14}H_{28}O_2$) montre que le pourcentage en ce produit augmente continuellement à mesure que l'intensité du courant augmente. Quant au pourcentage de l'hexène-1 non dégradé, il décroît très fortement lorsque l'intensité du courant augmente. Cette évolution est corroborée par les réactions d'aldocondensation qui se produisent au fur et à mesure que se forment les aldéhydes.

Pour de faibles intensités de courant ($I \leqq 20$ µA), on assiste à une augmentation du taux de formation de pentène-1. Lorsque l'intensité du courant augmente, le pourcentage de formation de pentène-1 diminue sensiblement.

Il ressort de cette analyse que l'échelle de l'intensité du courant peut être divisée en deux zones :

- une première zone ($I \leqq 50$ µA) où les pourcentages des produits "oxo" augmentent avec l'intensité du courant (zone de production) ;
- une deuxième zone ($I \geqq 50$ µA) où les pourcentages d'heptanol et d'heptanal diminuent pour voir augmenter le pourcentage d'aldocondensation (zone de "dégradation" des aldéhydes et des alcools). Cette différence de comportement des produits peut être exploitée pour améliorer le taux de formation des produits désirés.

On notera que les valeurs indiquées pour l'intensité du courant sont à adapter en fonction de la quantité de substrat et de la taille du réacteur entrant dans le procédé. En l'espèce, la quantité de substrat traité était dans nos expériences de 3 ml.

Le spectre IR de la figure 5 relatif aux variationx du courant électrique montre que l'amplitude des bandes d'absorption et des produits traités augmentent très fortement à mesure que l'intensité du courant augmente. En revanche, l'amplitude de la bande d'absorption à 1640 $cm^{-1}$ caractérisant la double liaison diminue très fortement lorsque l'intensité du courant augmente. L'augmentation de l'intensité de la bande à 1720 $cm^{-1}$ (C=O) et la diminution de la bande à 1640 $cm^{-1}$ (C=C) montrent que le groupe carboxylé se fixe sur la double liaison de l'alcène (hexène-1).

4) Influence de la distance interélectrode

La comparaison des spectres des figures 2 à 4 obtenus pour des distances interélectrodes égales à 7, 8, 10, 13 et 17 mm montre que l'intensité de ses bandes augmente en fonction de la distance interélectrode.

5) Influence du rapport $CO/H_2$ sur la nature des produits formés

L'étude de l'influence des débits relatifs en CO et $H_2$ sur la nature et les pourcentages des produits formés a été faite en maintenant l'intensité du courant constante (I est à 50 μA). L'analyse des produits liquides recueillis pendant les essais est effectuée par spectrométrie IR et par chromatographie.

Les mesures et les analyses ont permis de tracer les variations des taux d'hexène 1 non dégradé et ceux de l'heptanal-1, de l'heptanol-1, du laurate d'éthyle et du pentène 1 résultant de la dégradation de l'hexène 1 en fonction du rapport $CO/H_2$ (pour une même intensité), les résultats sont reportés au tableau 2 et sur la figure 7.

Le tableau 2 ci-après présente les conditions de travail et l'évolution des différents produits principaux obtenus en fonction du rapport $CO/H_2$

Tableau 2

| Conditions % X Produits formés | $CO/H_2$=2 X % produits | $CO/H_2$=1 X % produits | $CO/H_2$=1/2 X % produits | $CO/H_2$=1/3 X % produits | $CO/H_2$=1/4 X % produits |
|---|---|---|---|---|---|
| Pentène-1 | 1,62 | 1,50 | 1,61 | 1,91 | 1,63 |
| Hexène-1 | 1,40 | 2,16 | 8,38 | 11,60 | 14,10 |
| Heptanal-1 | 19,14 | 18,33 | 10,85 | 16,36 | 19,51 |
| Heptanol-1 | 19,83 | 17,56 | 12,13 | 13,25 | 16,80 |
| Ethyl-laurate | 20,54 | 21,45 | 25,32 | 23,11 | 22,76 |

Ces courbes indiquent que le pourcentage d'hexène-1 non dégradé décroît fortement lorsque le rapport $CO/H_2$ augmente. Le taux de pentène-1 formé reste constant quelle que soit la variation du rapport $CO/H_2$. Les taux de formation de l'heptanol-1 et de l'heptanal-1, présentent un minimum pour un rapport $CO/H_2$ = 1/2, puis croissent dans le même sens pour se stabiliser vers une valeur de 20 %. Parallèlement, la teneur en laurate d'éthyle augmente très fortement lorsque le rapport $CO/H_2$ augmente, passe par un maximum pour $CO/H_2$ = 1/2 puis diminue pour se stabiliser vers une valeur constante quelle que soit la variation du rapport (voir figure 8). Ainsi la valeur de $CO/H_2$ = 1/2 constitue un rapport permettant de rationaliser la synthèse des produits d'aldocondensation au détriment des alcools et des aldéhydes "oxo". La théorie montre que la réaction conduisant à l'obtention des produits d'aldocondensation est très rapide et peut se faire sans catalyseur, ceci confirme nos résultats. Notons toutefois que la discordance entre la thérorie et nos résultats semble être établie lorsque le rapport $CO/H_2$ = 1/2. Cet exemple mérite une attention particulière car il va à l'encontre de la théorie généralement admise selon laquelle l'augmentation du débit en hydrogène vis-à-vis de celui du monoxyde de carbone se traduit par une augmentation du pourcentage en alcool au détriment des aldéhydes. Les courbes relatives à l'heptanol-1 et l'heptanal-1 présentent un minimum pour $CO/H_2$ = 1/2 (voir figure 8). Le fait que, dans le cas présent, l'augmentation du débit d'hydrogène au lieu d'augmenter le pourcentage en alcools, le diminue au profit des produits d'aldocondensation, montre que ce paramètre est très insuffisant pour expliquer à lui

seul le pourcentage de formation d'alcools.

6) Influence de débits de CO et $H_2$

Pour préciser le rôle de chacun des réactifs CO et $H_2$, on a tracé pour une même intensité de courant (I = 50 μA), les variations des pourcentages d'hexène-1 non dégradé en fonction des débits de CO, de $H_2$ et de mélange CO + $H_2$. Ces résultats sont reportés sur la figure 9.

Ces courbes montrent que les pourcentages en hexène-1 non dégradé décroissent lorsque les débits en CO et $H_2$ augmentent. Cette décroissance est beaucoup plus prononcée en atmosphère de CO et dans un mélange CO + $H_2$ qu'en atmosphère de $H_2$. Ce faible pourcentage d'hexène-1 en atmosphère de $H_2$ par rapport aux atmosphères de CO pur et CO + $H_2$ peut être expliqué en termes de puissance électrique. En effet, l'augmentation du débit d'hydrogène à courant constant provoque une baisse de la tension de la décharge. C'est ainsi qu'en maintenant le courant constant (I = 50 μA), l'augmentation du débit d'hydrogène de 0,1 l/min à 0,30 l/min fait passer la tension de 28 kV à 16 kV, ce qui explique l'inefficacité de $H_2$ dans la dégradation de l'hexène-1 en décharge couronne.

La recherche de l'explication relative à la décroissance des alcools formés peut être examinée en termes de catalyse en phase hétérogène en général, et de la nature spécifique de la catalyse en phase plasma en particulier.

Sur la base de cette hypothèse, une étude parallèle par spectrométrie IR a permis de mettre en évidence (voir figures 10 et 11) la très forte augmentation des bandes d'absorption à 3404 $cm^{-1}$, 1683-1720 $cm^{-1}$ et 1280-1480 $cm^{-1}$.

Les spectres IR des figures 10 et 11 montrent que ces bandes augmentent très fortement lorsque le rapport $CO/H_2$ diminue, traduisant la formation d'alcools, d'acides carboxyliques ou d'ester et d'eau.

La présence d'eau montre que l'aldéhyde formé peut subir une aldolisation et une crotonisation pour donner des aldéhydes non saturés selon le schéma réactionnel suivant :

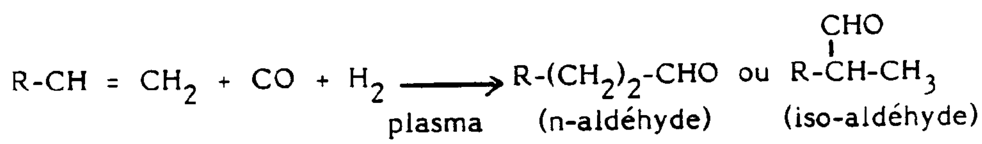


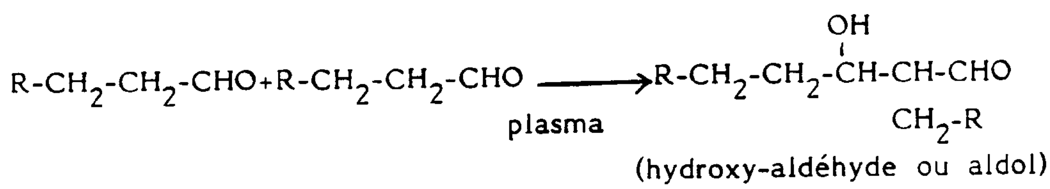


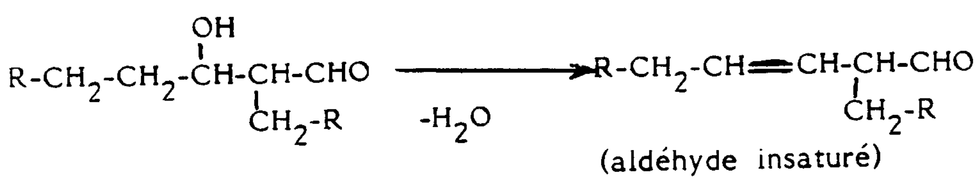


En conclusion, la diminution du rapport $CO/H_2$ accentue très fortement la formation des aldéhydes et alcools "oxo" qui se convertissent facilement en aldols.

7) Influence de la durée de la décharge sur les pourcentages des produits formés

L'étude de l'influence de traitement sur les pourcentages des produits a été effectuée pour un même rapport $CO/H_2$ = 1/2 et pour une même intensité de courant (I = 50 μA).

Afin de limiter les phénomènes de craquage et d'évaporation du substrat, nous avons effectué des essais de très courtes durées (essais de 5,8 et 1/2 minutes).

L'analyse des spectres IR des substrats traités montre que les bandes d'absorption à 3427 $cm^{-1}$ caractérisant les liaisons O-H et celles situées à 1683 $cm^{-1}$ (liaisons C = O) augmentent dans le même sens que la durée de traitement. On note toutefois une croissance très prononcée des bandes à 3427 $cm^{-1}$ au détriment

des bandes à 1682 $cm^{-1}$. Les spectres de la figure 12 relative à l'hexène-1 témoin et aux produits traités pendant 4 ; 8,39 et 12 minutes représentent les différentes variations.

L'ensemble de ces résultats montre l'importance de la durée de la décharge quant à l'obtention des différents produits.

En effet, le flux de CO activé vers le matériau dépend de la densité d'énergie des espèces relatives créées dans la décharge. Or, les deux processus (craquage et fixation de CO) dépendent très fortement de la durée de traitement du matériau par le plasma, c'est-à-dire des mécanismes réactionnels élémentaires qui conditionnent l'évolution globale des réactions ultérieures. Par conséquent, une durée minimale de traitement est nécessaire pour l'amorçage de la décharge et l'obtention d'un produit.

En particulier, pour une quantité de substrat de 3 ml, la durée minimale de traitement est de 4 minutes.

8) Installation avec un réacteur à plasma thermique et dispositif de trempe par lit fluidisé

La figure 13 est une vue schématique d'une installation avec dispositif de lit fluidisé réalisée conformément à une variante de l'invention.

Sur la figure 13, l'installation comprend un dispositif à lit fluidisé comportant une enceinte 1 de forme générale parallélépipédique dont le fond 2 qui présente une forme évasée vers le haut est raccordé au niveau de sa partie inférieure à des moyens d'injection d'un gaz de fluidisation.

Un réacteur tubulaire 3 est raccordé à la partie supérieure de l'enceinte 1 de telle façon que le réacteur 3 communique avec l'intérieur de l'enceinte et une torche à plasma 4 comportant de façon classique des inducteurs, traverse une paroi latérale de l'enceinte 1 de façon à injecter un plasma dans une masse M de particules disposée dans l'enceinte 1. Les particules de la masse M sont destinées à être fluidisées en un lit de type jaillissant par le gaz de fluidisation 7 pénétrant dans l'enceinte.

Les particules M par exemple particules d'$Al_2O_3$ fluidisées ont une granulométrie telle que 43 % en poids des billes ont un diamètre compris entre 500 et 350 µm et 57 % en poids ont un diamètre compris entre 630 et 500 µm. Cette proportion granulométrique permet une fluidisation jaillisante sans entraîner de particules dans l'enceinte 1 jusqu'à un débit du gaz de fluidisation de l'ordre de 50 l/min.

Le réacteur tubulaire 3 est constitué d'une colonne à garnissage fonctionnant selon le principe des colonnes d'absorption. La sortie du réacteur tubulaire 3 est reliée à des moyens de récupération et de fractionnement par condensation des produits hydrocarbonés sortants 6.

Ces moyens peuvent comprendre au voisinage de la sortie du réacteur tubulaire 3, un réfrigérant à eau auquel est relié successivement un piège à glace carbonique et un piège à azote liquide. En aval, le piège à azote liquide peut être relié à une fiole de lavage dans laquelle une dépression est engendrée par une pompe.

En service, le fonctionnement de l'installation qui vient d'être décrite est le suivant. La masse M de particules solides, de diamètre déterminé, sont mises en fluidisation en un lit jaillissant présentant la forme d'une fontaine en retombant vers les parois de l'enceinte par le débit constant d'un gaz de fluidisation consistant en de l'hydrogène.

La torche à plasma 4 injecte un plasma contenant un mélange $CO+H_2$ latéralement dans le lit de particules fluidisé où il transfère en se mélangeant et en se refroidissant une partie de sa chaleur aux particules qui échangent cette chaleur avec le gaz de fluidisation.

La réaction se produit dans le réacteur tubulaire 3. Les produits hydrocarbonés 5 à traiter circulant dans la colonne à contre courant du courant du mélange de réactifs actives.

Les produits hydrocarbonés 6 sortant du réacteur tubulaire 3 sont ensuite fractionnés en fonction de leur point de condensation dans le réfrigérant à eau, dans le piège à glace carbonique et dans le piège à azote liquide pour les produits les plus légers.

La torche à plasma fonctionne par exemple à une température de 5 MHz à une puissance allant jusqu'à 9 kW et un rendement de 50 %. Le débit de gaz plasmagène est par exemple de 40 à 50 l/min.

## Revendications

1. Procédé de synthèse de produits "oxo" ou leurs dérivés consistant en des composés hydrocarbonés présentant des groupes carbonyles et/ou hydroxyles, caractérisé en ce qu'on fait réagir un substrat hydrocarboné présentant une insaturation tel qu'un alcène avec des réactifs consistant en un mélange d'hydrogène et de monoxyde de carbone, la réaction ayant lieu suite à la mise en contact du substrat avec les espèces activées neutres du mélange de réactifs activé à l'état de plasma.

2. Procédé selon la revendication 1, caractérisé en ce qu'on fait réagir un alcène pour obtenir un mélange

comportant l'aldéhyde et l'alcool résultant de la fixation respectivement d'un groupe

$$H-\overset{|}{C}=O$$

ou de son groupe dérivé obtenu par réduction

$$H-\overset{|}{C}-OH$$

sur la double liaison de l'alcène, ou l'un seulement de ces deux composants en le séparant du mélange.

3. Procédé selon l'une des revendications précédentes, caractérisé en ce que l'activation du mélange hydrogène-monoxyde de carbone se fait dans un réacteur à plasma, le substrat étant mis en contact avec le mélange activé en dehors de la zone d'activation du réacteur.

4. Procédé selon la revendication 3, caractérisé en ce que le substrat et les réactifs sont introduits dans le réacteur à la pression atmosphérique et à une température voisine de la température ambiante.

5. Procédé selon l'une des revendications précédentes, caractérisé en ce que le rapport des débits volumiques $CO/H_2$ est supérieur à 1.

6. Procédé selon l'une des revendications précédentes, caractérisé en ce que le rapport des débits volumiques $CO/H_2$ est de l'ordre de 1/2.

7. Procédé selon l'une des revendications 3 à 6, caractérisé en ce que le réacteur à plasma est du type à décharge couronne pointe-plan, la pointe étant disposée parallèlement à l'électrode plane et le substrat est introduit dans le réacteur dans l'axe de la pointe en dessous de la zone d'activation laquelle se situe entre la pointe et le plan.

8. Procédé selon la revendication 7, caractérisé en ce que l'électrode pointe est chargée positivement et l'électrode plane est reliée à la masse.

9. Procédé selon l'une des revendications 7 ou 8, caractérisé en ce que le monoxyde de carbone est introduit au niveau de l'électrode pointe et l'hydrogène est introduit à la périphérie du réacteur.

10. Procédé selon l'une des revendications 1 à 6, caractérisé en ce que le réacteur à plasma est du type à plasma thermique, le mélange $CO+H_2$ activé étant, après activation, soumis à une trempe par lit de particules fluidisé par un courant gazeux d'hydrogène à température de 20 à 150°C avant d'être mis en contact avec le substrat à traiter.

11. Installation utile dans le procédé selon l'une des revendications 1 à 9, consistant en un réacteur à plasma du type décharge couronne pointe-plan, caractérisée en ce qu'il comporte une ou plusieurs électrodes pointes creuses par lesquelles le réacteur est alimenté en gaz de monoxyde de carbone, l'électrode pointe étant disposée parallèlement à l'électrode plane, l'hydrogène étant introduit à la périphérie du réacteur et le substrat hydrocarboné liquide étant placé sous la pointe dans son axe à une distance pointe substrat inférieure à la distance pointe plan.

12. Installation utile pour la mise en oeuvre du procédé selon la revendication 10, caractérisé en ce qu'elle comprend un réacteur à plasma haute fréquence produisant une torche plasma $CO+H_2$ injectée latéralement dans une enceinte d'un dispositif à lit fluidisé, un réacteur tubulaire du type colonne à garnissage étant relié à la sortie de ladite enceinte, dans lequel le substrat circule à contre courant du flux de mélange $CO+H_2$ activé.

**Patentansprüche**

1. Verfahren zur Synthese von "Oxo"-Produkten oder ihren Derivaten, die bestehen aus Kohlenwasserstoffverbindungen, die Carbonyl- und/oder Hydroxylgruppen aufweisen, dadurch gekennzeichnet, daß man ein eine Unsättigung aufweisendes Kohlenwasserstoff-Substrat wie ein Alken mit Reagentien reagieren läßt, die aus einer Mischung von Wasserstoff und Kohlenmonoxid bestehen, wobei die Reaktion stattfindet nach dem Kontaktieren des Substrats mit den neutralen aktivierten Molekülen der Reagensmischung, die im Plasma-Zustand aktiviert worden ist.

2. Verfahren nach Anspruch 1, dadurch gekennzeichnet, daß man ein Alken reagieren läßt unter Bildung einer Mischung aus einem Aldehyd und einem Alkohol, die resultieren aus der jeweiligen Bindung einer

H-C=O-Gruppe

oder einer durch Reduktion davon abgeleiteten

H-C-OH-Gruppe

an die Doppelbindung des Alkens oder nur einer dieser beiden Bestandteile, den man von der Mischung abtrennt.

3. Verfahren nach einem der vorhergehenden Ansprüche, dadurch gekennzeichnet, daß die Aktivierung des Wasserstoff/Kohlenmonoxid-Gemisches in einem Plasma-Reaktor erfolgt, wobei das Substrat außerhalb der Aktivierungszone des Reaktors mit der aktivierten Mischung in Kontakt gebracht wird.

4. Verfahren nach Anspruch 3, dadurch gekennzeichnet, daß das Substrat und die Reagentien bei Atmosphärendruck und einer Temperatur in der Nähe von Umgebungstemperatur in den Reaktor eingeführt werden.

5. Verfahren nach einem der vorhergehenden Ansprüche, dadurch gekennzeichnet, daß das Volumen-Durchflußverhältnis $CO/H_2$ oberhalb 1 liegt.

6. Verfahren nach einem der vorhergehenden Ansprüche, dadurch gekennzeichnet, daß das Volumen-Durchflußverhältnis $CO/H_2$ in der Größenordnung von 1/2 liegt.

7. Verfahren nach einem der Ansprüche 3 bis 6, dadurch gekennzeichnet, daß der Plasma-Reaktor ein solcher vom Spitzen-Flächen-Coronaentladungs-Typ ist, wobei die Spitze parallel zur ebenen Elektrode angeordnet ist und das Substrat in der Achse der Spitze unterhalb der Aktivierungszone, die zwischen der Spitze und der Fläche (Ebene) liegt, eingeführt wird.

8. Verfahren nach Anspruch 7, dadurch gekennzeichnet, daß die spitze Elektrode positiv aufgeladen wird und daß die ebene Elektrode geerdet wird.

9. Verfahren nach einem der Ansprüche 7 oder 8, dadurch gekennzeichnet, daß das Kohlenmonoxid in Höhe der spitzen Elektrode eingeführt wird und daß der Wasserstoff in die Peripherie (den Umfang) des Reaktors eingeführt wird.

10. Verfahren nach einem der Ansprüche 1 bis 6, dadurch gekennzeichnet, daß der Plasma-Reaktor ein solcher vom thermischen Plasma-Typ ist, in dem die aktivierte Mischung $CO+H_2$ nach der Aktivierung einer Abschreckung in einem Bett aus aufgewirbelten Teilchen mit einem Wasserstoffgasstrom einer Temperatur von 20 bis 150°C unterworfen wird, bevor er mit dem zu behandelnden Substrat in Kontakt gebracht wird.

11. Vorrichtung zur Durchführung des Verfahrens nach einem der Ansprüche 1 bis 9, die besteht aus einem Plasma-Reaktor vom Spitzen-Flächen-Coronaentladungs-Typ, dadurch gekennzeichnet, daß sie eine oder mehrere hohle spitze Elektroden aufweist, durch welche der Reaktor mit Kohlenmonoxidgas beschickt wird, wobei die spitze Elektrode parallel zu der ebenen Elektrode angeordnet ist, der Wasserstoff in die Peripherie des Reaktors eingeführt wird und das flüssige Kohlenwasserstoff-Substrat unter der Spitze in ihrer Achse in einem bestand Spitze-Substrat angeordnet ist, der kleiner ist als der bestand Spitze-Fläche.

12. Vorrichtung zur Durchführung des Verfahrens nach Anspruch 10, dadurch gekennzeichnet, daß sie umfaßt einen Hochfrequenz-Plasma-Reaktor, der einen seitlich in einen Behälter einer Wirbelbett-Anordnung injizierten Plasmastrahl aus $CO+H_2$ erzeugt, wobei ein rohrförmiger Reaktor vom Säulen-Typ mit einer Auskleidung mit dem Ausgang des genannten Behälters verbunden ist, in dem das Substrat im Gegenstrom zu dem aktivierten $CO+H_2$-Mischungsstrom zirkuliert.

## Claims

1. Process for the synthesis of "oxo" products or their derivatives consisting of hydrocarbon compounds containing carbonyl and/or hydroxyl groups, characterized in that a hydrocarbon substrate containing an unsaturation, such as an alkene, is reacted with reactants consisting of a mixture of hydrogen and carbon monoxide, the reaction taking place after bringing the substrate into contact with the neutral activated species of the mixture of reactants activated to the plasma state.

2. Process according to Claim 1, characterized in that an alkene is reacted to obtain a mixture containing the aldehyde and the alcohol resulting from the fixation on the double bond of the alkene of, respectively, a group

$$H-\overset{|}{C}=O$$

or its derivated group

$$H-\overset{|}{C}-OH$$

obtained by reduction, or only one of these two components by separating it from the mixture.

3. Process according to one of the preceding claims, characterized in that the activation of the hydrogen/carbon monoxide mixture takes place in a plasma reactor, the substrate being brought into contact with the activated mixture outside the activation zone of the plasma.

4. Process according to Claim 3, characterized in that the substrate and the reactants are introduced into the reactor at atmospheric pressure and at a temperature close to ambient temperature.

5. Process according to one of the preceding claims, characterized in that the ratio of the $CO/H_2$ volume flow rates is greater than 1.

6. Process according to one of the preceding claims, characterized in that the ratio of the $CO/H_2$ volume flow rates is of the order of 1/2.

7. Process according to one of Claims 3 to 6, characterized in that the plasma reactor is of the point-plane corona discharge type, the point being arranged parallel to the plane electrode, and the substrate is introduced into the reactor in the axis of the point and below the activation zone, which is located between the point and the plane.

8. Process according to Claim 7, characterized in that the point electrode is positively charged and the plane electrode is connected to earth.

9. Process according to one of Claims 7 or 8, characterized in that the carbon monoxide is introduced at the level of the point electrode and the hydrogen is introduced at the periphery of the reactor.

10. Process according to one of Claims 1 to 6, characterized in that the plasma reactor is of the thermal plasma type, the activated $CO+H_2$ mixture being subjected, after activation, to chilling by a fluidized bed of particles by a stream of hydrogen gas at a temperature of 20 to 150°C before being brought into contact with the substrate to be treated.

11. Installation, usable in the process according to one of Claims 1 to 9, consisting of a plasma reactor of the point-plane corona discharge type, characterized in that it comprises one or more hollow point electrodes through which the reactor is fed with carbon monoxide gas, the point electrode being arranged parallel to the plane electrode, the hydrogen being introduced at the periphery of the reactor and the liquid hydrocarbon substrate being placed under the point in its axis at a point-substrate distance smaller than the point-plane distance.

12. Installation, usable for carrying out the process according to Claim 10, characterized in that it comprises a high-frequency plasma reactor producing a $CO+H_2$ plasma torch injected laterally into a chamber of a fluidized bed apparatus, a tubular reactor of the packing column type being connected to the outlet of the said chamber, in which the substrate circulates in counter-current to the flow of activated $CO+H_2$ mixture.

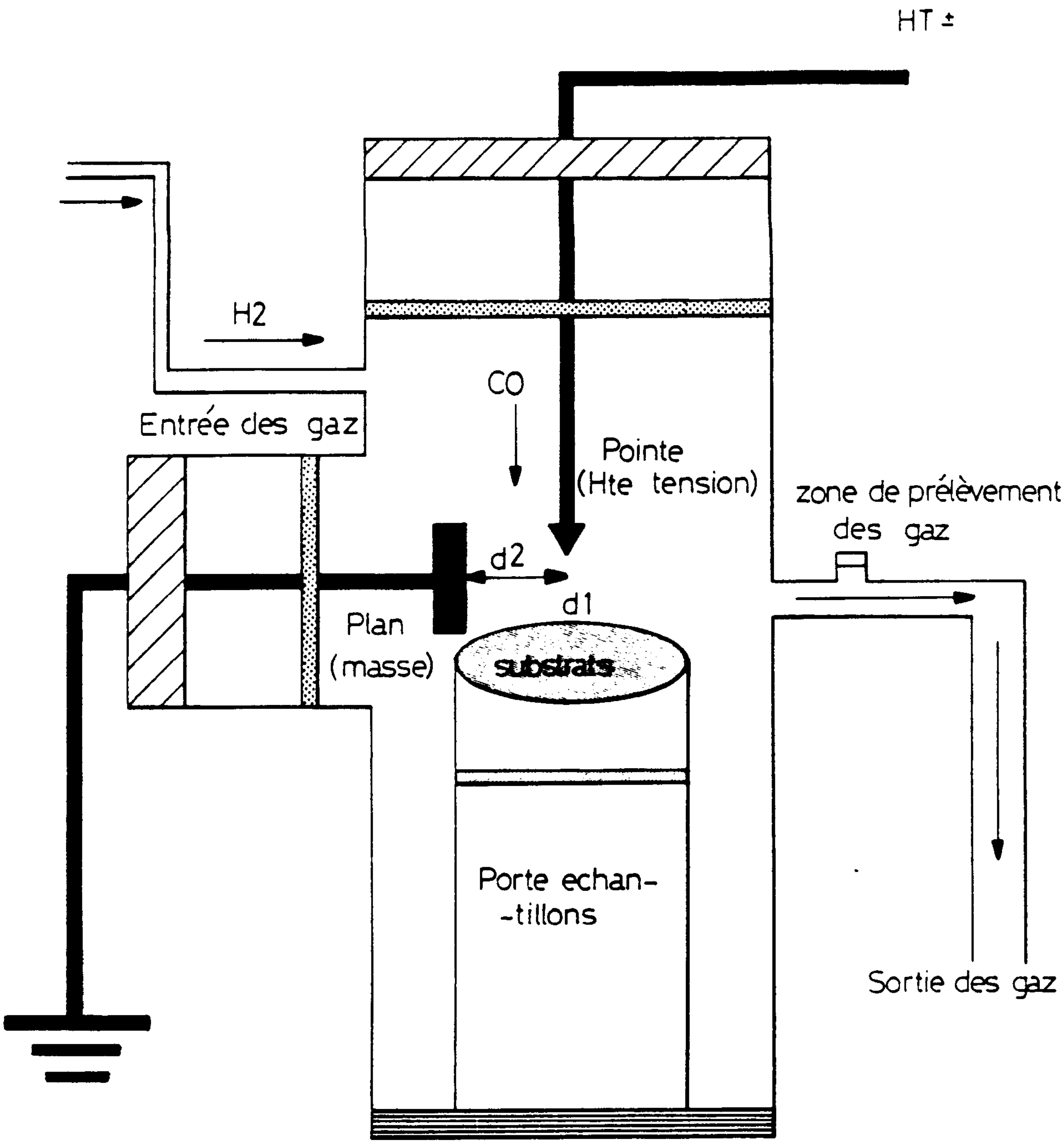
HT ±
H2
Entrée des gaz
CO
Pointe
(Hte tension)
zone de prélèvement
des gaz
d2
d1
Plan
(masse)
substrats
Porte echan-
-tillons
Sortie des gaz

FIG_1

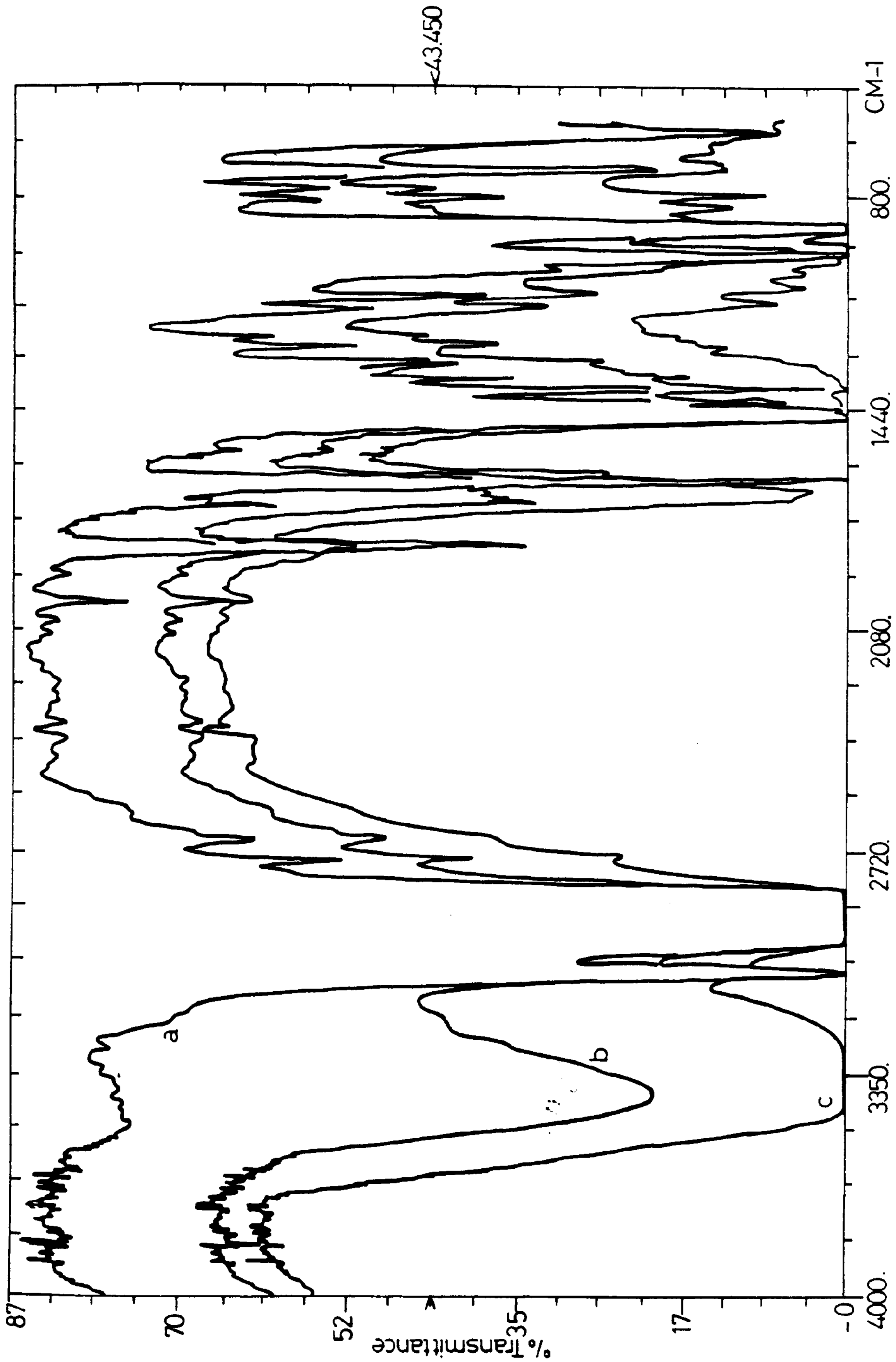
87
70
52
35
17
-0
% Transmittance
4000.
3350.
2720.
2080.
1440.
800.
CM-1
<43.450
a
b
c

FIG_2

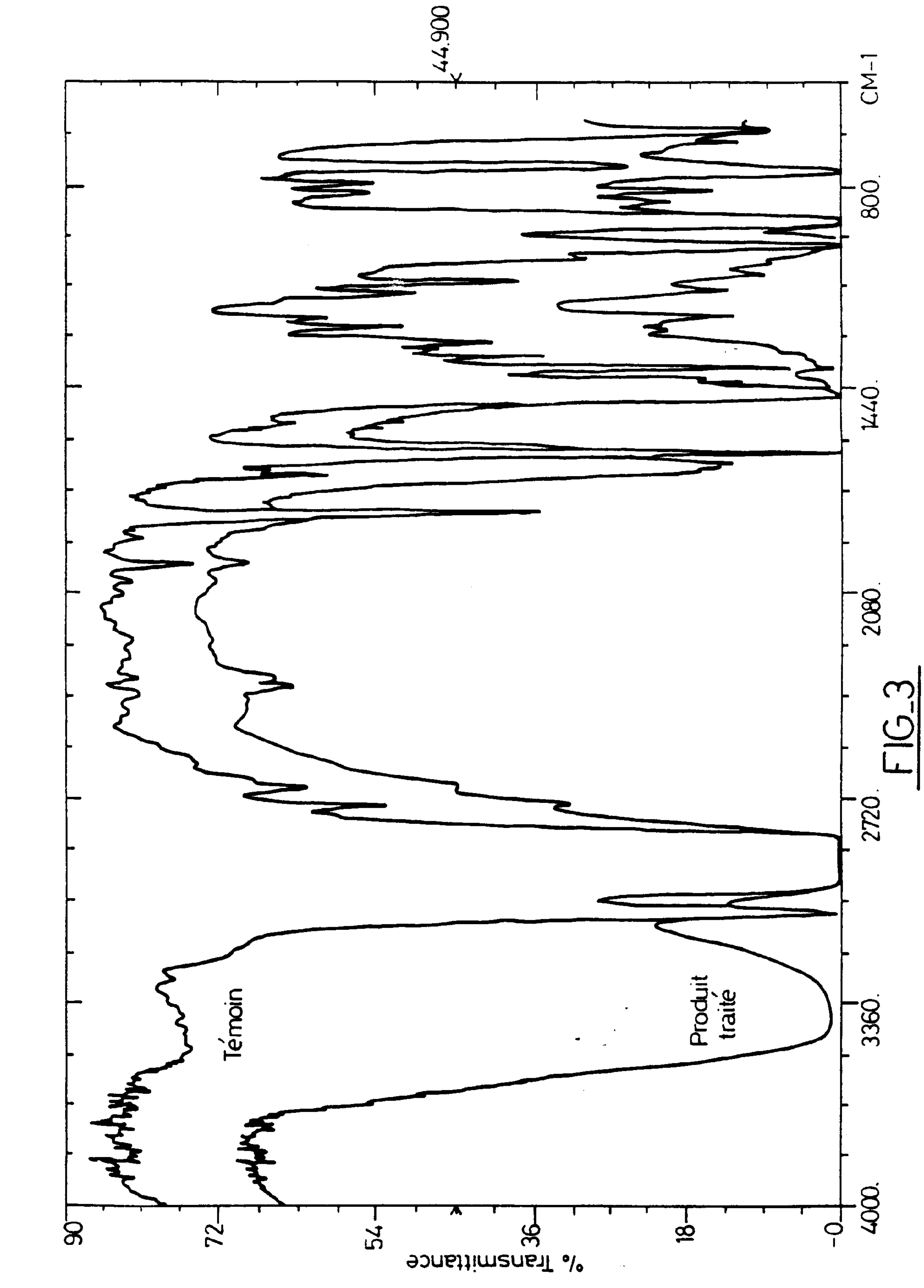
44.900
CM-1
800.
1440.
2080.
2720.
3360.
4000.
Témoin
Produit traité
90
72
54
36
18
0
% Transmittance

FIG_3

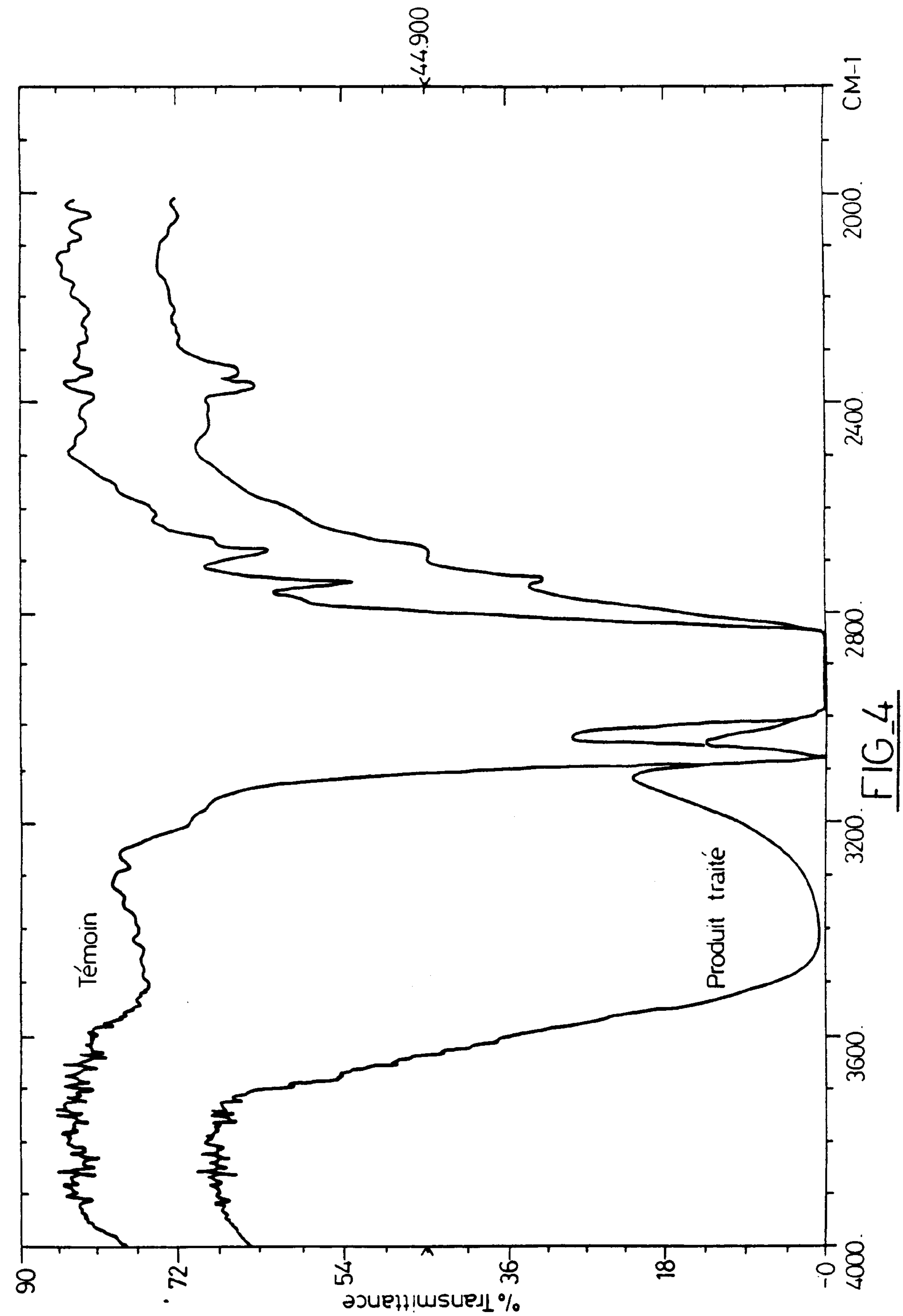

44.900
CM-1
2000
2400
2800
3200
3600
4000
90
72
54
36
18
0
% Transmittance
Témoin
Produit traité


FIG_4

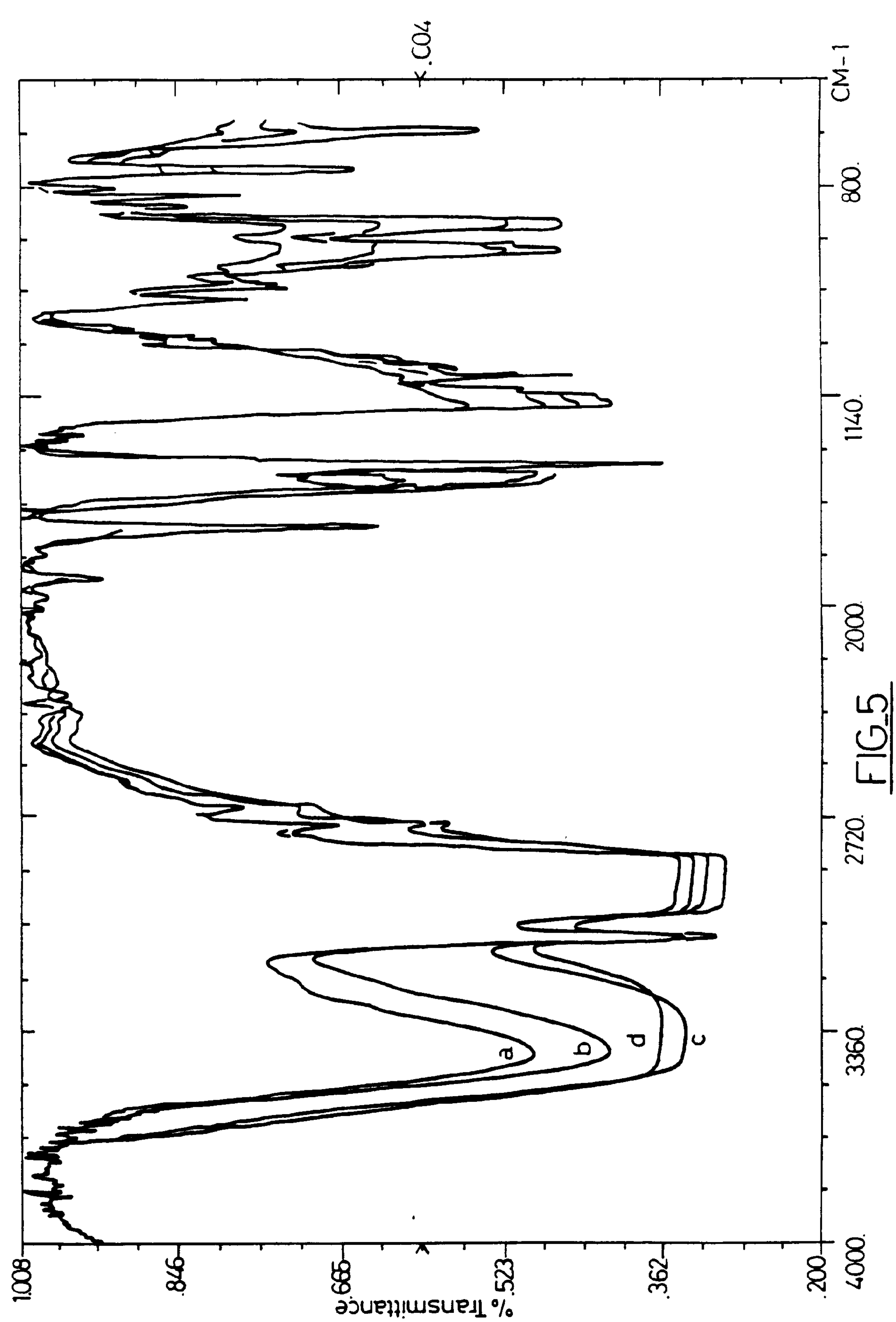
% Transmittance
1.008
.846
.685
.523
.362
.200
4000
3360
2720
2000
1140
800
CM-1
a
b
c
d
.C04

FIG_5

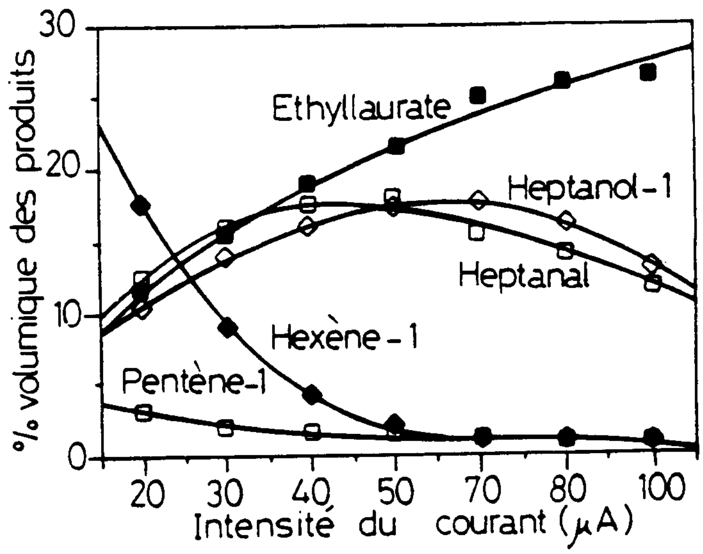
30
20
10
0
% volumique des produits
Ethyllaurate
Heptanol-1
Heptanal
Hexène-1
Pentène-1
20
30
40
50
70
80
100
Intensité du courant (μA)

FIG_6

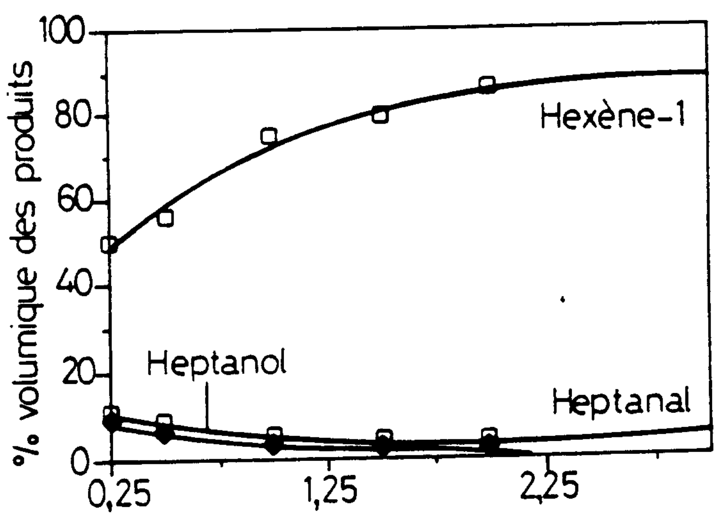
100
80
60
40
20
0
% volumique des produits
Hexène-1
Heptanol
Heptanal
0,25
1,25
2,25

FIG_7

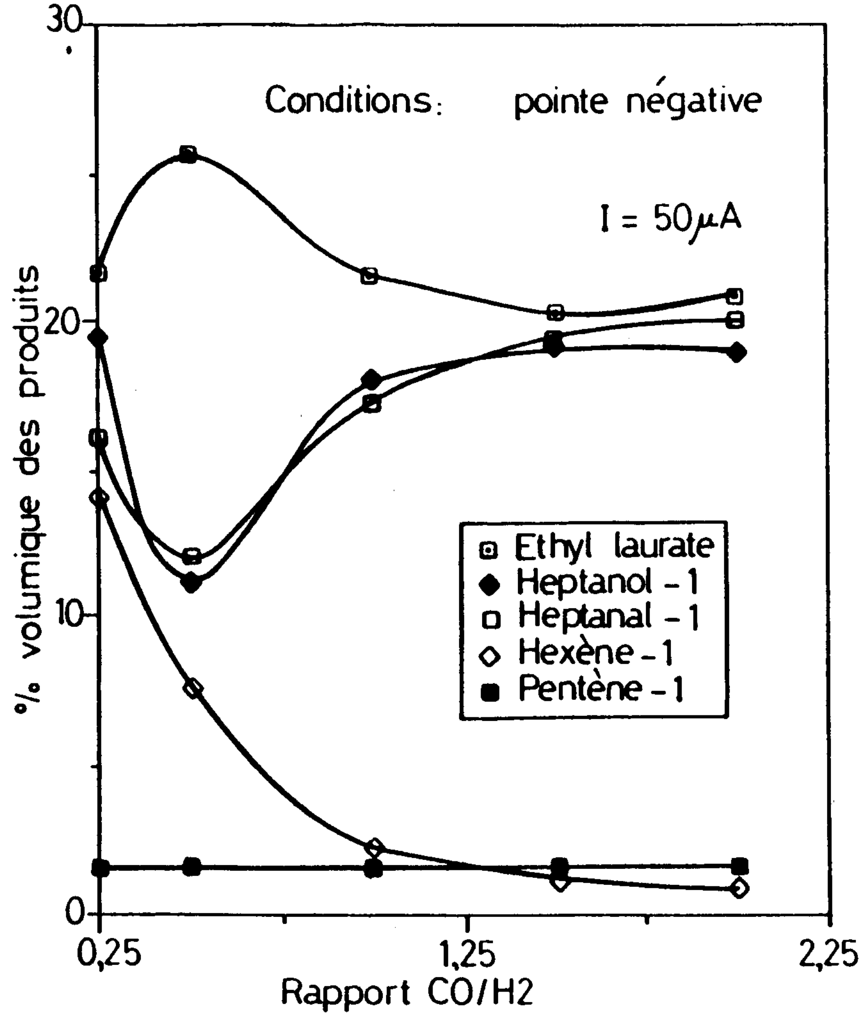
30
20
10
0
0,25
1,25
2,25
Rapport CO/H2
% volumique des produits
Conditions: pointe négative
I = 50μA
Ethyl laurate
Heptanol - 1
Heptanal - 1
Hexène - 1
Pentène - 1

FIG_8

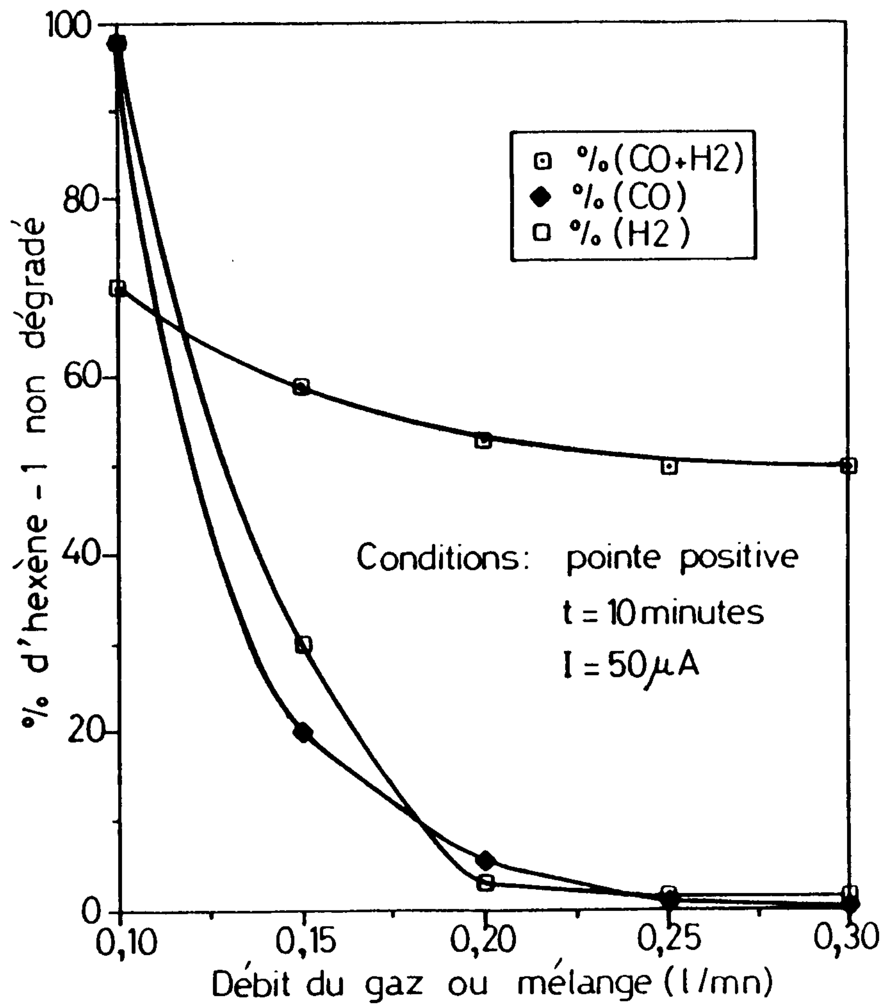
100
80
60
40
20
0
0,10
0,15
0,20
0,25
0,30
Débit du gaz ou mélange (l/mn)
% d'hexène - 1 non dégradé
%(CO+H2)
%(CO)
%(H2)
Conditions: pointe positive
t = 10 minutes
I = 50μA

FIG_9

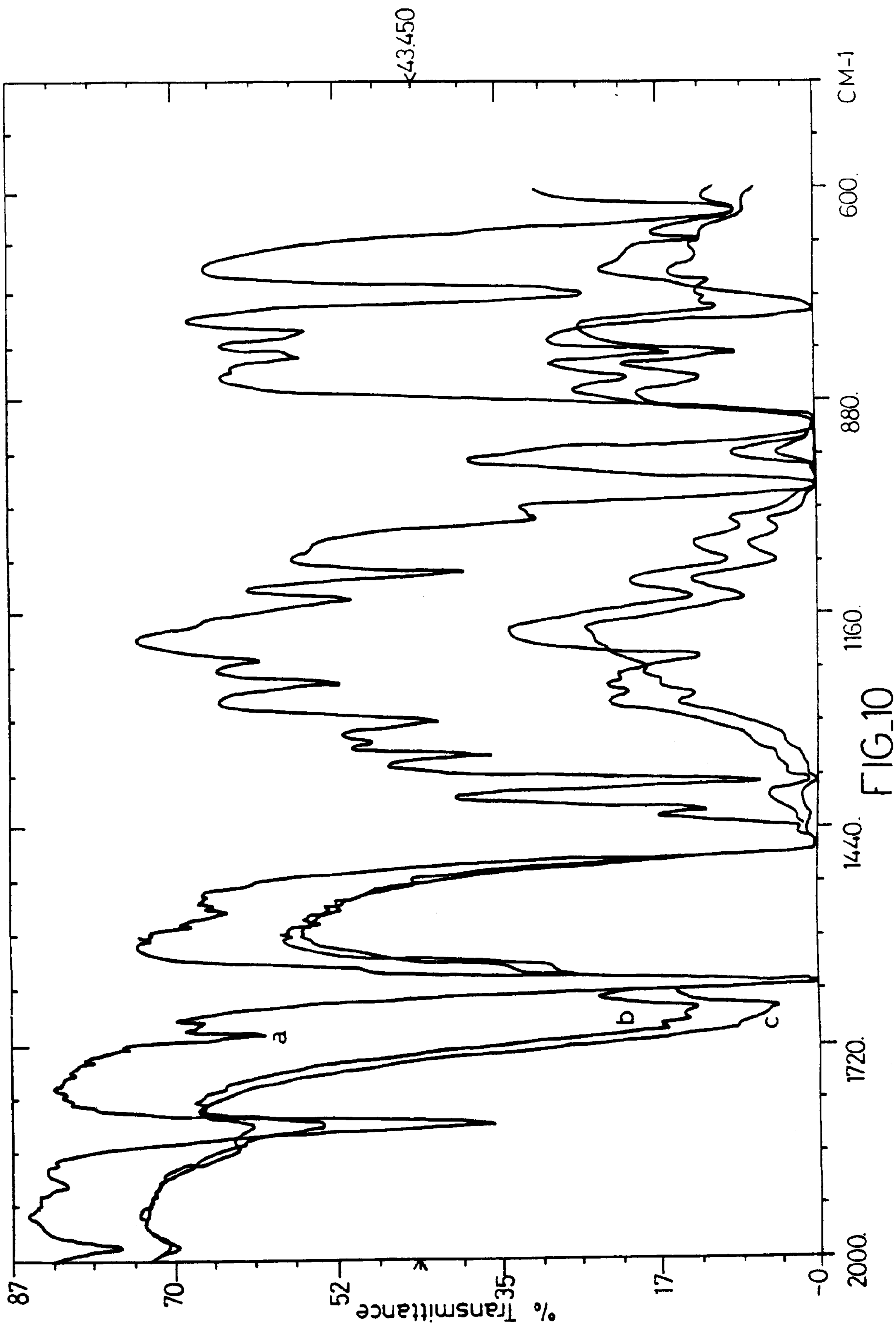
43.450
CM-1
600.
880.
1160.
1440.
1720.
2000.
87
70
52
35
17
0
% Transmittance
a
b
c

FIG_10

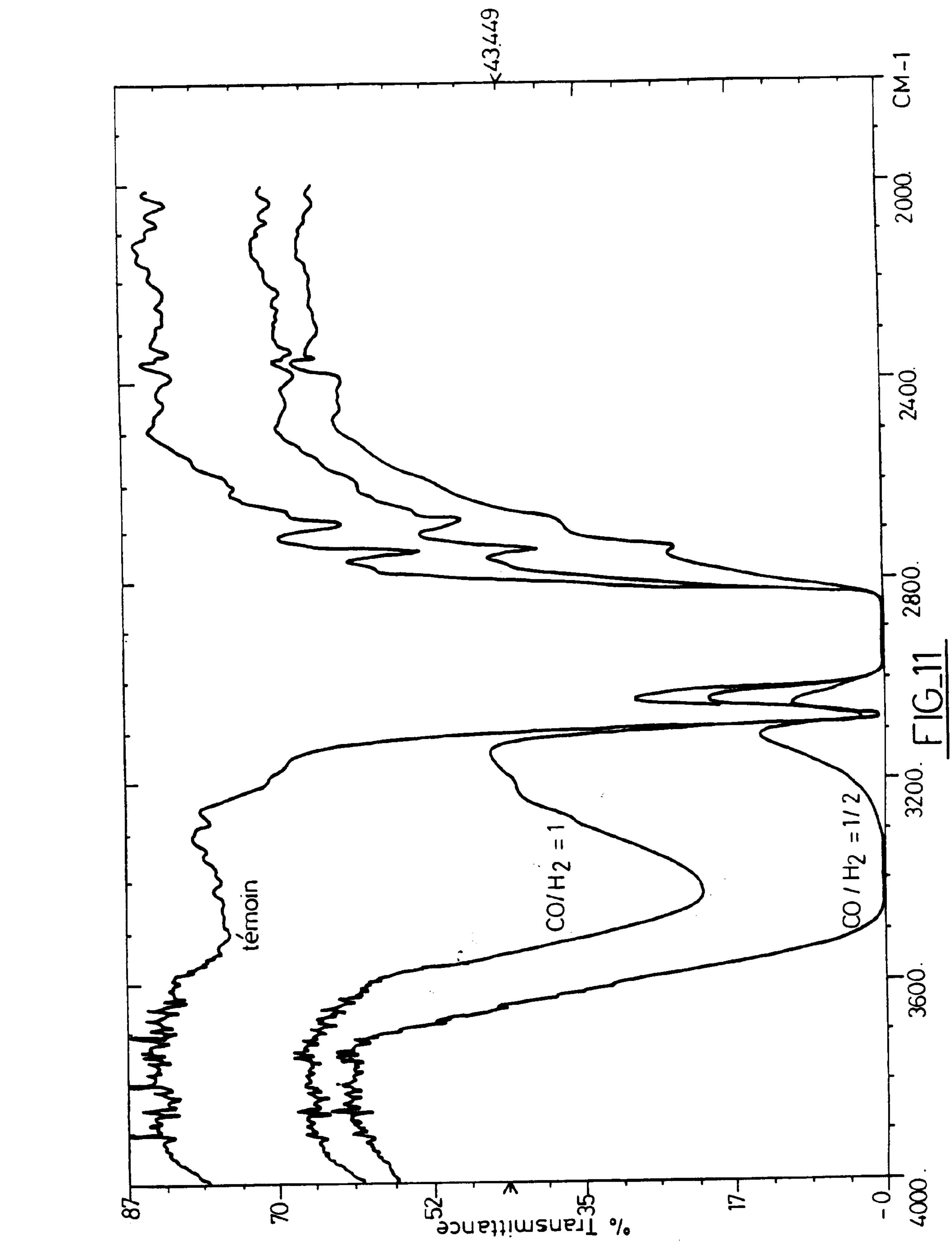

% Transmittance
87
70
52
35
17
-0
4000
3600
3200
2800
2400
2000
CM-1
<43449
témoin
CO/H2 = 1
CO / H2 = 1/ 2


FIG_11

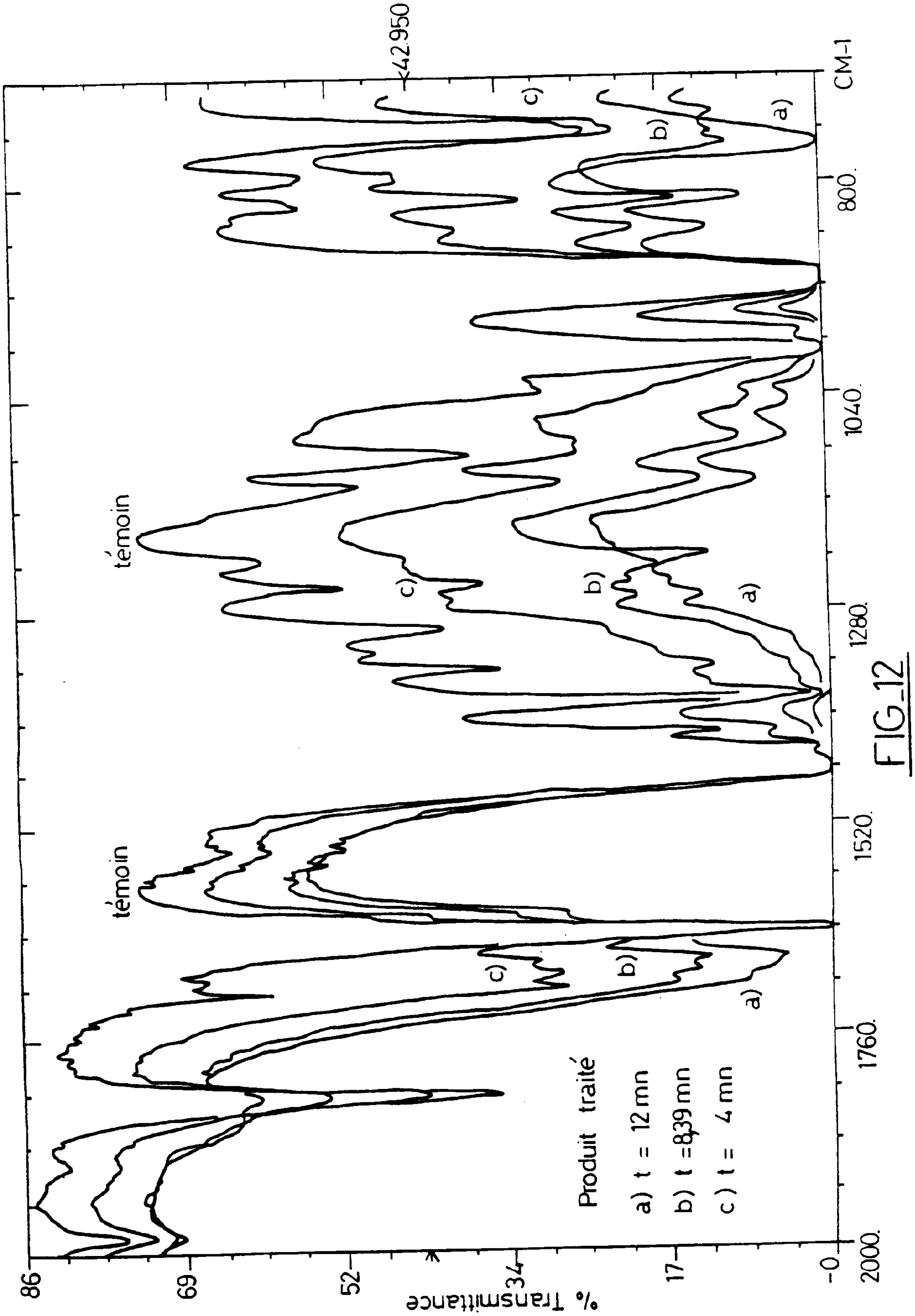
% Transmittance
86
69
52
34
17
0
2000.
1760.
1520.
1280.
1040.
800.
CM-1
42.950
témoin
témoin
a)
b)
c)
Produit traité
a) t = 12 mn
b) t = 8,39 mn
c) t = 4 mn

FIG_12

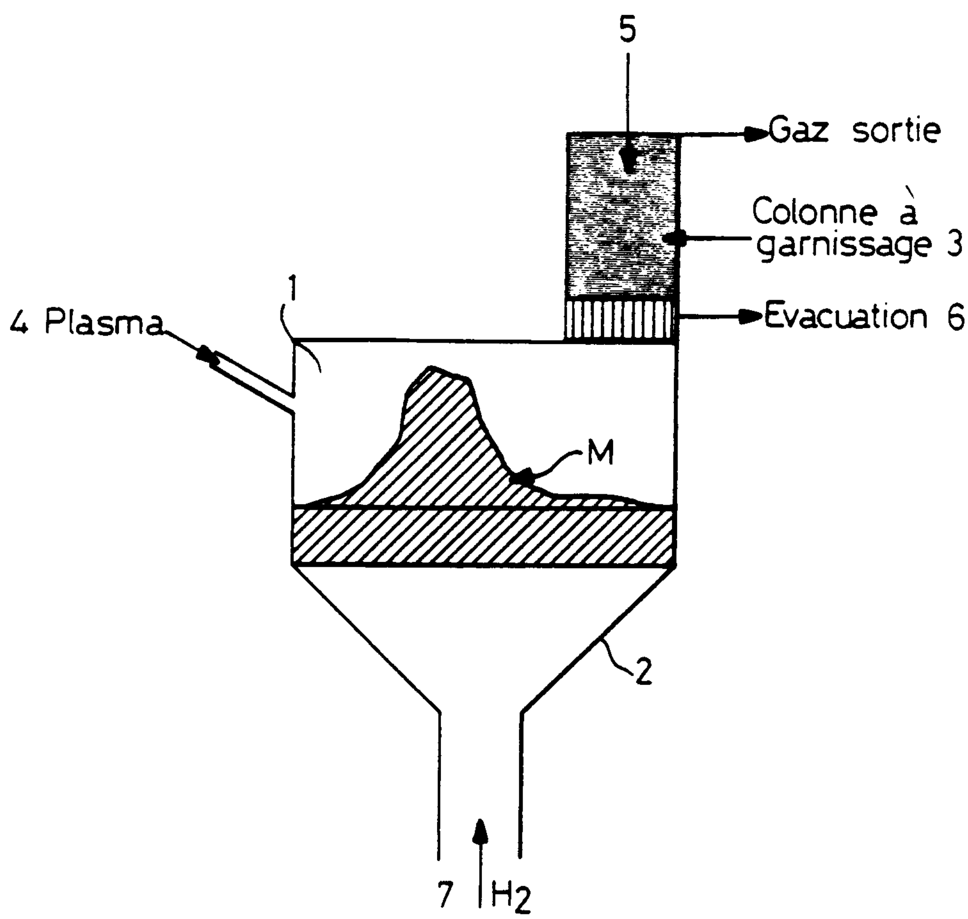
5
Gaz sortie
Colonne à garnissage 3
Evacuation 6
1
4 Plasma
M
2
7
H2

FIG_13